# EUROPÄISCHE PATENTSCHRIFT

(11) **EP 4 523 525 B1**
(45) Veröffentlichungstag und Bekanntmachung des Hinweises auf die Patenterteilung: **18.11.2026**
(21) Anmeldenummer: 23197144.1
(22) Anmeldetag: 13.09.2023
(51) Int. Cl.: A01K 67/36, A01K 67/366

(54) **INSEKTENZUCHT MIT VORDOSIERTEN EINHEITEN**
INSECT BREEDING WITH PRE-DOSED UNITS
ÉLEVAGE D'INSECTES AVEC UNITÉS PRÉ-DOSEES

(43) Veröffentlichungstag der Anmeldung: 19.03.2025
(73) Patentinhaber: Livin Farms Agrifood GmbH, 1110 Wien (AT)
(72) Erfinder: Unger, Katharina, 1120 Wien (AT)
(74) Vertreter: Weiss Patentanwalts GmbH

(56) Entgegenhaltungen:
- WO-A1-2021/148894
- TW-U- M 609 282
- US-A1- 2003 188 698

## Beschreibung

Die gegenständliche Erfindung betrifft ein Verfahren zum Einfüllen von Insekten mit einer Dosiervorrichtung in zumindest einen Behälter zur Insektenzucht, wobei die Insekten in einem gleichen bestimmten Wachstumsstadium, insbesondere als Larven, oder in unterschiedlichen bestimmten Wachstumsstadien sind, wobei der zumindest eine Behälter mit einem Substrat gefüllt ist, welches vorgegebene Eigenschaften aufweist, um ein Wachstum der Insekten in dem zumindest einen Behälter zu fördern, wobei die Dosiervorrichtung zumindest eine Lagereinheit umfasst, welche dazu ausgebildet ist, die Insekten zu lagern.

Ein Lebenszyklus eines Insekts beschreibt verschiedene Wachstumsstadien (beispielsweise Ei, Larve, Puppe und erwachsenes Insekt), wobei die Dauer dieser Wachstumsstadien und die Wachstumsstadien je nach Art des Insekts variieren. Der Begriff "Insekt" umfasst dabei das Insekt in den verschiedenen Wachstumsstadien. Je nach Art der Insekten können die Wachstumsphasen einige Tage bis einige Wochen in Anspruch nehmen. In der industriellen Insektenzucht werden die Insekten insbesondere für Lebensmittel und für Tierfutter gezüchtet. Von der Produktion der Insekteneier bis hin zum Heranzüchten der erwachsenen Insekten, deckt die industrielle Insektenzucht die verschiedenen Wachstumsstadien der Insekten ab. Nachdem die Insekten als Larven aus den Eiern schlüpfen, werden diese üblicherweise in mit Substrat gefüllten Behältern oder Boxen in vorgegebene Umgebungsbedingungen gelagert, wo diese zu erwachsenen Insekten heranwachsen. Während der Lagerung in den Behältern können sich die Larven auch zur Puppe verpuppen, woraus die erwachsenen Insekten heranwachsen. Das Substrat umfasst dabei Futter für die Larven. Die erwachsenen Insekten werden "geerntet", also aus den Behältern entfernt, und können anschließend, z.B. zu proteinreichen Endprodukten wie Öl oder Pulver, weiterverarbeitet werden. Vergleichbar mit anderen Branchen werden Produktionsanlagen in der industriellen Insektenzucht ebenfalls großteils automatisiert betrieben.

In WO 2022/144197 A1, in US 2018/0070566 A1 oder in CN 115968837 A sind Layouts solcher Produktionsanlagen offenbart. Dabei werden in verschiedenen Stationen die Insekten als Larven zuerst in die mit Substrat gefüllten Behälter gefüllt, anschließend werden die Behälter eingelagert und nach einer gewissen Wachstumsperiode wieder ausgelagert und gegebenenfalls entleert (z.B., wenn die Insekten von den Larven zu erwachsenen Insekten herangewachsen sind). Die Insekten als Larven können entweder gemeinsam mit dem Substrat in die Behälter gefüllt werden oder jeweils separat. Die Behälter können dabei auch in vorgegebenen Abständen aus- und, beispielsweise nach einer Kontrolle oder nach Hinzufügen von weiterem Substrat, wieder eingelagert werden. In US 2016/0066552 A1 wird der Inhalt der Behälter mittels Kameras kontrolliert, wobei eine Dosiervorrichtung dazu dient zusätzliches Substrat in die Behälter zu füllen. Dieses Aus- und Einlagern erhöht den Aufwand während der Insektenzucht.

Für das Einfüllen der Insekten als Larven und/oder des Substrats in die Behälter sind üblicherweise Dosiervorrichtung vorgesehen, welche eine Wiegeeinrichtung umfassen. Die Wiegeeinrichtung dient dazu, nach dem Einfüllen zu kontrollieren, ob eine vorgegebene Menge (Dosierung) an Larven und/oder Substrat im jeweiligen Behälter vorhanden ist. In WO 2022/144197 A1 oder in WO 2022/048792 A1 ist eine Dosiervorrichtung offenbart, wobei durch Wiegen überprüft wird, ob genügend Larven und/oder genügend Substrat in den Behältern vorhanden ist. Neben dem höheren Aufwand durch die Wiegeeinrichtung verlangsamt das Wiegen zudem die Produktion.

US 2003/188698 A1 betrifft Roboteranlagen und Verfahren zur Haltung von Beständen kleiner Organismen, wie beispielsweise Fruchtfliegen oder verschiedener anderer kleiner Organismen, die für die Erforschung der Genetik von Nutzen sind. Die Organismen werden mithilfe einer Transferstation umgesetzt. Die die Organismen enthaltenden Röhrchen werden mithilfe eines Roboterarms transportiert.

Grundsätzlich ist die Menge an Insekten als Larven vom Substrat im Behälter abhängig. Das Substrat weist dabei Eigenschaften wie Material, Menge, Dichte, Feuchtigkeit, Nährstoffgehalt, usw., auf. Je nachdem welches Substrat mit welchen Eigenschaften im Behälter vorhanden ist, ist eine dementsprechende Menge an Larven zu dosieren, um beispielsweise eine vorgegebene Dichte der Larven (Anzahl der Larven pro Volumeneinheit) und des Substrats im Behälter zu erhalten, um einen bestmöglichen Ertrag in der Insektenzucht zu erhalten. Natürlich ist diese Dichte auch vom Behältervolumen abhängig. Die Dichte der Larven und das Substrat im Behälter beeinflusst dabei das Wachstum der Insekten. Hierfür gibt es für einen bestmöglichen Zuchterfolg einen bekannten Zusammenhang zwischen dem Substrat im Behälter und der Dichte der Insekten im Behälter.

Es ist daher eine Aufgabe der gegenständlichen Erfindung die Insektenzucht zu verbessern, insbesondere die Genauigkeit der Dosierung der Insekten im Behälter, beispielsweise in Abhängigkeit von einem Substrat im Behälter, zu erhöhen und somit das Wachstum der Insekten im Behälter und den Zuchterfolg zu verbessern.

Erfindungsgemäß wird die Aufgabe durch die Merkmale des Hauptanspruchs gelöst, wobei bei Verwendung der Dosiervorrichtung eine vorgegebenen Menge an Insekten in einer Verpackung verpackt ist und in der zumindest einen Lagereinheit gelagert ist, wobei die vorgegebene Menge der Insekten in einem bekannten Verhältnis auf die vorgegebenen Eigenschaften des Substrats im zumindest einen Behälter abgestimmt ist, und wobei die Dosiervorrichtung eine Robotereinheit umfasst, welche im Bereich der zumindest einen Lagereinheit und des zumindest einen Behälters angeordnet ist und dazu ausgebildet ist, bei Verwendung der Dosiervorrichtung die Verpackung mit den Insekten in der vorgegebenen Menge aus der zumindest einen Lagereinheit zu entnehmen und die Verpackung mit den Insekten in der vorgegebenen Menge zum zumindest einen Behälter zu bewegen und die Robotereinheit dazu ausgebildet ist, die Insekten in der vorgegebene Menge aus der Verpackung in das Substrat des zumindest einen Behälters direkt oder indirekt zu füllen. Dadurch ergibt sich eine genauere Dosierung der Insekten in Abhängigkeit von den vorgegebene Eigenschaftes des Substrats, wodurch folglich ein besseres Wachstum der Insekten in dem zumindest einen Behälter ermöglicht wird. Insbesondere ist es damit einfach möglich, das Substrat und dessen bekannten Eigenschaften und die Menge an in den Behälter zu füllenden Insekten einfach, sicher und reproduzierbar aufeinander abzustimmen. Auf Grund des sich dadurch ergebenden besseren Wachstums der Insekten in dem zumindest einen Behälter verringern sich Ausfälle in der Insektenzucht und der Zuchterfolg erhöht sich. Durch die Robotereinheit entfällt zudem ein manuelles Einfüllen der Insekten in den zumindest einen Behälter, wodurch sich einerseits der Arbeitsaufwand reduziert und sich andererseits das Einfüllen automatisieren lässt.

Vorteilhafterweise ist die Verpackung bei Verwendung der Dosiervorrichtung zusätzlich zu der vorgegebenen Menge an Insekten mit einem Substrat gefüllt ist, wobei das Substrat vorgegebene Eigenschaften aufweist. Dabei kann einerseits sichergestellt werden, dass für die Insekten ausreichend Feuchtigkeit, Nährstoffe, usw., vorhanden sind, um deren Überleben in der Verpackung für eine gewisse Zeit zu sichern. Andererseits können Reststoffe aus der Insektenzucht, wie beispielsweise Exkremente von Insekten oder bereits verwendetes Substrat, weiterverwertet werden.

Vorteilhafterweise ist die vorgegebene Menge an Insekten und das Substrat in der Verpackung gleichmäßig vermengt sind. Durch die homogene Aufteilung des Substrats und der Insekten in der Verpackung kann auch eine homogene Aufteilung beim und nach dem Einfüllen in die Behälter erreicht werden. Damit kann ein eventuell ansonsten notwendiges Homogenisieren von Substrat und Insekten im Behälter unterbleiben.

In einer bevorzugten Anordnung ist die erfindungsgemäße Dosiervorrichtung im Bereich eines Fördersystem angeordnet, wobei der zumindest eine Behälter auf dem Fördersystem angeordnet ist. In der Insektenzucht werden auf Grund des hohen logistischen Aufwandes die Behälter bevorzugt auf Fördersystemen befördert. Somit ist das Einfüllen der Insekten in die Behälter auf dem Fördersystem besonders effizient und auch bei bestehenden Fördersystemen einfach zu implementieren.

Beim erfindungsgemäßen Verfahren zum Einfüllen von Insekten in zumindest einen Behälter mit der Dosiervorrichtung sind die Insekten in einer vorgegebenen Menge in einer Verpackung verpackt und die Verpackung wird mit den Insekten in der vorgegebenen Menge in der zumindest einen Lagereinheit gelagert, wobei die vorgegebene Menge der Insekten in einem bekannten Verhältnis auf die vorgegebenen Eigenschaften des Substrats im zumindest einen Behälter abgestimmt ist, wobei die Verpackung mit den Insekten in der vorgegebenen Menge von einer Robotereinheit aus der zumindest einen Lagereinheit entnommen wird und die Robotereinheit die Verpackung mit den Insekten in der vorgegebenen Menge zum zumindest einen Behälter bewegt und die Robotereinheit die Insekten in der vorgegebene Menge aus der Verpackung in das Substrat des zumindest einen Behälters direkt oder indirekt füllt. Auf Grund der vorgegebenen Menge an Insekten in Abhängigkeit von den vorgegebene Eigenschaftes des Substrats, erhöht sich die Genauigkeit der Dosierung der Insekten. Folglich wird ein besseres Wachstum der Insekten in dem zumindest einen Behälter ermöglicht, wodurch sich Ausfälle in der Insektenzucht verringern und sich die Ausbeute erhöht. Durch die Robotereinheit kann auf ein manuelles Einfüllen der Insekten in den zumindest einen Behälter verzichtet werden, wodurch sich einerseits der Arbeitsaufwand reduziert und andererseits das Einfüllen automatisierter abläuft.

Erfindungsgemäß füllt die Robotereinheit die Insekten in der vorgegebenen Menge aus der Verpackung direkt in das Substrat des zumindest einen Behälters, indem die Robotereinheit die Verpackung öffnet und die Insekten in der vorgegebene Menge in den zumindest einen Behälter füllt. Dadurch kann die Verpackung nach dem Einfüllen der Insekten in den zumindest einen Behälter beispielsweise an der Dosiervorrichtung entsorgt werden. Das Entfernen der Verpackung aus dem zumindest einen Behälter in einem weiteren Schritt der Insektenzucht, beispielsweise nach der Lagerung des zumindest einen Behälters, entfällt.

Alternativ füllt die Robotereinheit die Insekten in der vorgegebenen Menge indirekt in das Substrat des zumindest einen Behälters, indem die Robotereinheit die Verpackung mit den Insekten in der vorgegebenen Menge in den zumindest einen Behälter legt. Dies ist insbesondere z.B. bei einer biologisch abbaubaren Verpackung vorteilhaft, da sich die Verpackung in dem zumindest einen Behälter zersetzt, und kein Entfernen der Verpackung aus dem zumindest einen Behälter notwendig ist. Damit können auch Verpackungen verwendet werden, durch die sich die Insekten durchfressen können.

Bevorzugt wird bevor oder gleichzeitig oder nachdem die Insekten in der vorgegebenen Menge in den zumindest einen Behälter gefüllt werden, der zumindest eine Behälter mit dem Substrat befüllt wird. Insbesondere bei gleichzeitigem Einfüllen verringert sich der Arbeitsaufwand, da ein separates Einfüllen der Insekten und des Substrats entfällt. Vorteilhafterweise füllt die Robotereinheit den zumindest einen Behälter mit dem Substrat. Somit verringert sich der technische Aufwand für das Einfüllen der Insekten, da die Robotereinheit sowohl das Einfüllen der Insekten als auch das Einfüllen des Substrats in den zumindest einen Behälter durchführen kann.

Die gegenständliche Erfindung wird nachfolgend unter Bezugnahme auf die Figuren 1 bis 4 näher erläutert, die beispielhaft, schematisch und nicht einschränkend vorteilhafte Ausgestaltungen der Erfindung zeigen. Dabei zeigt
Fig. 1 den grundlegenden Aufbau der erfindungsgemäßen Dosiervorrichtung,
Fig. 2 ein beispielhaftes Verfahren zum Einfüllen von Insekten mit der erfindungsgemäßen Dosiervorrichtung,
Fig. 3a ein beispielhaftes direktes Einfüllen der Insekten,
Fig. 3b ein beispielhaftes indirektes Einfüllen der Insekten, und
Fig. 4 eine bevorzugte Anordnung der erfindungsgemäßen Dosiervorrichtung.

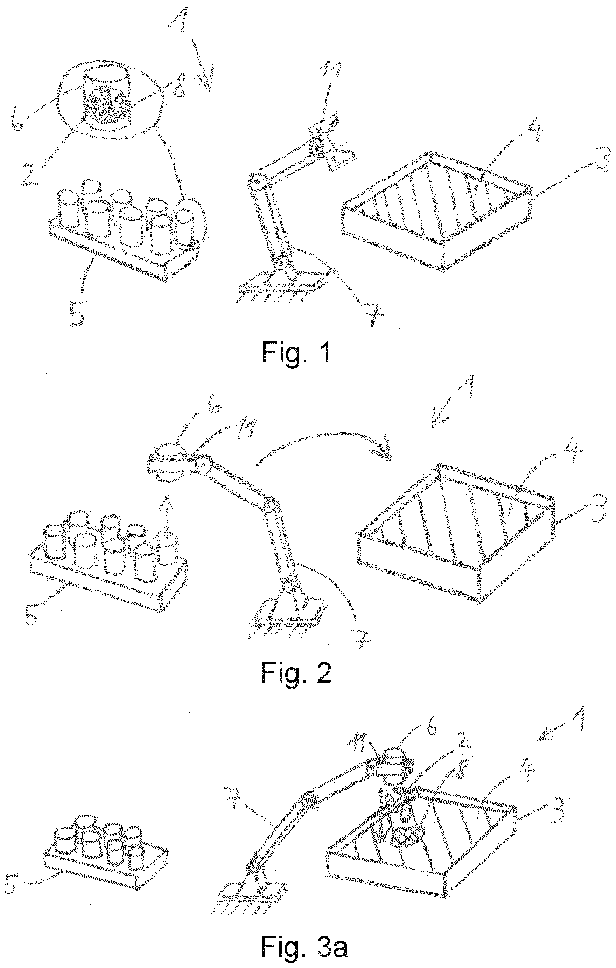

In Fig. 1 ist der grundlegende Aufbau der erfindungsgemäßen Dosiervorrichtung 1 zum Einfüllen von Insekten 2 in zumindest einen Behälter 3 zur Insektenzucht dargestellt. Die Dosiervorrichtung 1 umfasst eine Robotereinheit 7 und zumindest eine Lagereinheit 5, wobei die zumindest eine Lagereinheit 5 im Arbeitsbereich der Robotereinheit 7 angeordnet ist und dazu ausgebildet ist, die Insekten 2 zu lagern.

Eine Robotereinheit 7 ist allgemein ein ein- oder mehrachsiger Manipulator (z.B. ein Roboterarm) mit zumindest einem Greifer 11, zum Greifen und Halten eines Gegenstandes, insbesondere einer Verpackung 6. Die Robotereinheit 7 hat einen Arbeitsbereich, in dem sich der Greifer 11 bewegen kann, um z.B. eine "Pick and Place" Aufgabe durchzuführen. Die Robotereinheit 7 ist vorzugsweise als Industrieroboter ausgeführt. Die Robotereinheit 7 umfasst auch eine Robotersteuerung, mittels der die Bewegung der Robotereinheit 7, insbesondere des Greifers 11 gesteuert wird. Weiters kann die Robotereinheit 7 auch Sensoren umfassen, welche z.B. zur Steuerung der Robotereinheit 7 dienen.

Die Insekten 2, die in einen Behälter 3 zu füllen sind, sind in einem bestimmten Wachstumsstadium. Dabei können die Insekten 2 alle im gleichen Wachstumsstadium, insbesondere als Larven (wie in Fig. 1 dargestellt) oder als Eier, Puppen usw., sein oder in unterschiedlichen bestimmten Wachstumsstadien sein. Die Insekten 2 können dabei insbesondere jene Arten von Insekten 2 umfassen, welche zur Insektenzucht geeignet sind (z.B. Larven einer schwarzen Soldatenfliege).

In Fig. 1 ist ein Behälter 3 dargestellt, wobei natürlich eine Mehrzahl an Behältern 3 für das Einfüllen von Insekten 2 vorgesehen werden kann. Der zumindest eine Behälter 3 kann in unterschiedlichen Formen und Materialien ausgestaltet sein, wie z.B. in Fig. 1 dargestellt als nach oben hin offene, rechteckförmige Kiste oder Box, mit vier Seitenwänden und einer Bodenplatte jeweils aus Kunststoff. Der Behälter 3 kann auch mehrere abgetrennte Kammern aufweisen. Der zumindest eine Behälter 3 dient, neben der Aufnahme von Insekten 2, zur Aufnahme eines Substrats 4, welches vorgegebene Eigenschaften (wie etwa Material, Menge, Dichte, Feuchtigkeit, Nährstoffgehalt, usw.) aufweist, um ein Wachstum der Insekten 2 in dem zumindest einen Behälter 3 zu fördern. Als Substrat 4 eignet sich beispielsweise Gemüse, Getreide, Früchte, Stärke oder ähnliches.

Bei Verwendung der Dosiervorrichtung 1 ist eine vorgegebene Menge an Insekten 2 in einer Verpackung 6 verpackt und in der zumindest einen Lagereinheit 5 gelagert. Die vorgegebene Menge der Insekten 2 ist in einem bekannten Verhältnis auf die vorgegebenen Eigenschaften des Substrats 4 im zumindest einen Behälter 3 abgestimmt. Beispielsweise kann je nach Kundenwunsch eine Dichte der Insekten 2 (Anzahl der Insekten 2 pro Volumeneinheit) und die Menge des Substrats 4 im zumindest einen Behälter 3 vorgegeben sein, wobei dementsprechend die Menge der Insekten 2 auf die vorgegebenen Eigenschaften des Substrats 4 (z.B. ebenfalls durch Kunden vorgegeben) abgestimmt ist.

In Fig. 1 ist beispielhaft eine Mehrzahl an Verpackungen 6 in einer Lagereinheit 5 dargestellt, wobei eine Verpackung 6 lediglich zur Veranschaulichung vergrößert und für einen Blick in die Verpackung 6 teilweise aufgebrochen dargestellt ist. Dabei sind die Verpackungen 6 beispielhaft mit einer zylindrischen Form (z.B. als ein Becher) ausgeführt. Natürlich ist die Form der Verpackung 6 nicht darauf beschränkt und kann insbesondere auch als Beutel, Schachtel usw. ausgeführt sein. Die Verpackung 6 kann auch unterteilt sein in Verpackungseinheiten, z.B. ein Becher mit einer Mehrzahl an Kammern in denen die Insekten 2 gefüllt sind. Die Verpackung 6 kann aus unterschiedlichen Materialien ausgeführt sein, z.B. Kunststoff, Papier, Karton, etc. Dabei kann die Verpackung 2 auch beispielsweise biologisch abbaubar ausgeführt sein, z.B. aus einem biologisch abbaubaren Kunststoff, Papier, Karton, etc. Weiters ist die Verpackung 6 vorzugsweise geschlossen, z.B. ein Becher mit Deckel oder durch das Siegeln eines Kunststoffes, um ein Austreten der Insekten 2 in der Lagereinheit 5 zu verhindern. Die Verpackung 6 kann aber auch offen sein, z.B. ein Becher ohne Deckel.

Zusätzlich zu den Insekten 2 kann die Verpackung 6, wie in Fig. 1 dargestellt, auch mit einem Substrat 8 gefüllt sein, wobei das Substrat 8 in der Verpackung 6 vorgegebene Eigenschaften aufweist. Das Substrat 8 in der Verpackung 6 kann dabei gleiche oder unterschiedliche Eigenschaften zu jenem Substrat 3 im Behälter 4 haben. Das Substrat 8 schafft für die Insekten 2 in der Verpackung 6 Bedingungen (z.B. ausreichend Feuchtigkeit, Futter, Nährstoffe, etc.), damit die Insekten 2 auch bei einer Lagerung der Verpackung 6 über einen gewissen Zeitraum, wie mehrere Tage oder Wochen, überleben. Als Substrat 8 eignet sich beispielsweise Gelatine, Agartine (Agar-Agar), Getreidemischungen oder ähnliches. Das Substrat 8 in der Verpackung 6 kann aber auch Reststoffe aus der Insektenzucht umfassen, wie beispielsweise Exkremente von Insekten 2 oder bereits verwendetes Substrat. Das Substrat 8 und die Insekten 2 werden vorzugsweise vor dem Verpacken in die Verpackung 6 durch Mischen gleichmäßig vermengt (auch als Homogenisieren bezeichnet). Hierbei sind insbesondere die Menge der Insekten 2 in der Verpackung 6 und die vorgegebenen Eigenschaften des Substrats 8, wie Menge, Dichte, Feuchtigkeit, Nährstoffgehalt, usw., aufeinander abgestimmt.

In Fig. 1 ist eine Lagereinheit 5 dargestellt, wobei auch eine Mehrzahl an Lagereinheiten 5 vorgesehen werden kann. Eine Lagereinheit 5 hat zumindest eine Verpackungsaufnahme zur Aufnahme einer Verpackung 6 mit Insekten 2. Dabei kann die Lagereinheit 5 unterschiedlich ausgeführt sein. In Fig. 1 ist z.B. eine Kiste dargestellt, wobei darin die Mehrzahl an Verpackungen 6 rasterförmig angeordnet ist. Die Lagereinheit 5 kann auch stapelbar ausgeführt sein, um eine Mehrzahl an Lagereinheiten 5 zu stapeln. In der Lagereinheit 5 können zur Lagerung der Verpackung 6 vorgegebene Umgebungsbedingungen herrschen, z.B. kann die Lagereinheit 5 oder die Umgebung der Lagereinheit 5 temperiert sein, um die Insekten 2 in den Verpackungen 6 zur Lagerung auf einer bestimmten Temperatur zu halten.

Die Robotereinheit 7 ist im Bereich der zumindest einen Lagereinheit 5 und des zumindest einen Behälters 3 angeordnet ist, sodass der Arbeitsbereich der Robotereinheit 7 die Lagereinheit 5 und den Behälter 3 zumindest teilweise umfasst. Die Robotereinheit 7 könnte hierzu auch selbstfahrend ausgeführt sein und entlang des Bodens bewegbar sein. Die Robotereinheit 7 kann beispielsweise auch auf einer bewegbaren Plattform befestigt sein, um sich zwischen der Lagereinheit 5 und dem Behälter 3 zu bewegen.

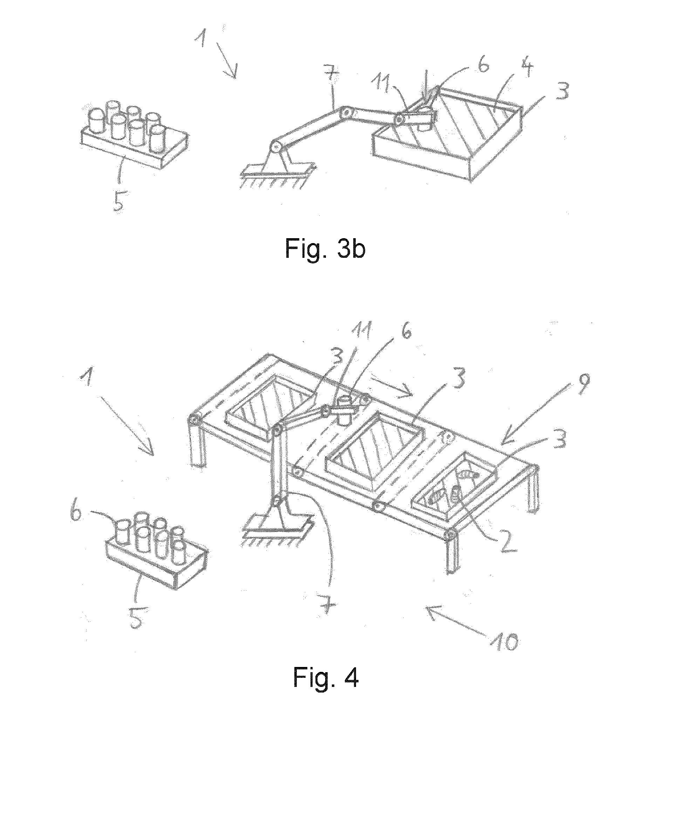

Die Robotereinheit 7 ist dazu ausgebildet, die Verpackung 6 mit den Insekten 2 in der vorgegebenen Menge aus der Lagereinheit 5 zu entnehmen und die Verpackung 6 mit den Insekten 2 in der vorgegebenen Menge zum Behälter 3 zu bewegen. Die Robotereinheit 7 ist weiters dazu ausgebildet, die Insekten 2 in der vorgegebenen Menge aus der Verpackung 6 in das Substrat 4 des Behälters 3 direkt oder indirekt zu füllen (in Fig. 3a und 3b dargestellt). Dabei weist die Robotereinheit 7, wie in Fig. 1 dargestellt, vorzugsweise zumindest einen Greifer 11 auf.

In Fig. 2 ist ein beispielhaftes Verfahren zum Einfüllen der Insekten 2 in den Behälter 3 mit dem grundlegenden Aufbau der erfindungsgemäßen Dosiervorrichtung 1 dargestellt. Der Behälter 3 wird vorzugsweise bevor die Insekten 2 in der vorgegebenen Menge in den Behälter 3 gefüllt werden, mit dem Substrat 4 befüllt. Weiters kann der Behälter 3 aber auch gleichzeitig und/oder nachdem die Insekten 2 in der vorgegebenen Menge gefüllt wurden, mit dem Substrat 4 befüllt werden. Dazu kann die Dosiervorrichtung 1 eine Füllvorrichtung (nicht dargestellt) umfassen, welche das Substrat 4, z.B. mittels eines Trogschneckenförderers oder ähnlichem, in den Behälter 3 füllt. Vorzugsweise wird das Substrat 4 jedoch von der Robotereinheit 7 in den Behälter 3 gefüllt. Die Robotereinheit 7 kann auch dazu ausgebildet sein, die Füllvorrichtung (z.B. mit der Robotersteuerung) zu steuern, um das Substrat 4 in den Behälter 3 zu füllen.

In Fig. 2 ist der Behälter 3 bereits mit Substrat 4 gefüllt. Die Robotereinheit 7 entnimmt daraufhin eine Verpackung 6 mit den Insekten 2 in der vorgegebenen Menge aus der Lagereinheit 5, z.B. durch Greifen und Anheben mit dem Greifer. Es ist auch möglich, dass die Robotereinheit 7 gleichzeitig eine Mehrzahl an Verpackungen 6 aus der Lagereinheit 5 entnimmt. Die Lagereinheit 5 kann beispielsweise auch ein Fördersystem (nicht dargestellt) umfassen, mit welchem die Verpackungen 6 aus der Lagereinheit 5 zu der Robotereinheit 7 gefördert werden, wobei die Robotereinheit 7 die Verpackungen 6 von dem Fördersystem entnimmt. Anschließend bewegt die Robotereinheit 7 die Verpackung 6 mit den Insekten 2 in der vorgegebenen Menge zum Behälter 3 (mit einem Pfeil angedeutet), damit die Robotereinheit 7 die Insekten 2 in der vorgegebenen Menge aus der Verpackung 6 in das Substrat 4 des Behälters 3 direkt oder indirekt einfüllen kann.

In Fig. 3a ist ein beispielhaftes direktes Einfüllen der Insekten 2 in der vorgegebenen Menge aus der Verpackung 6 in das Substrat 4 des Behälters 3 dargestellt. "Direkt" bedeutet in diesem Zusammenhang, dass die Robotereinheit 7 die Verpackung 6 öffnet (sofern die Verpackung 6 geschlossen ist) und die Insekten 2 in der vorgegebenen Menge in den Behälter 3 füllt. Die Verpackung 6 wird von der Robotereinheit 7, beispielsweise durch Aufstanzen, Aufschneiden, etc., geöffnet, wodurch die Insekten 2 aus der Verpackung 6 entleert werden können und in das Substrat 4 des Behälters 3 gelangen. Bei einer offenen Verpackung 6, z.B. einem Becher ohne Deckel, kann die Robotereinheit 7 die Verpackung 6 einfach kippen oder drehen, um die Insekten 2 aus der Verpackung 6 zu entleeren, damit die Insekten 2 in das Substrat 4 des Behälters 3 gelangen. Wie in Fig. 3a dargestellt, würde auch das sich in der Verpackung 6 befindliche Substrat 8 aus der Verpackung 6 in das Substrat 4 des Behälters 3 gelangen. Nachdem die Verpackung 6 entleert wurde, kann diese beispielsweise als Abfall von der Robotereinheit 7 entsorgt werden.

In Fig. 3b ist ein beispielhaftes indirektes Einfüllen der Insekten 2 in der vorgegebenen Menge aus der Verpackung 6 in das Substrat 4 des Behälters 3 dargestellt. "Indirekt" bedeutet in diesem Zusammenhang, dass die Verpackung 6 zum Einfüllen der Insekten 2 in der vorgegebenen Menge von der Robotereinheit 7 in den Behälter 3 abgelegt wird. Die Verpackung 6 kann ebenfalls von der Robotereinheit 7 zuerst geöffnet werden, wobei jedoch die Verpackung 6 mit den Insekten 2 in den Behälter 3 abgelegt wird. Es ist auch möglich, dass sich die Verpackung 6, z.B. wenn diese aus einem biologisch abbaubaren Material ausgeführt ist, in dem Behälter 3 zersetzt und sich dadurch zumindest teilweise öffnet. Es wäre ebenfalls möglich, dass die Verpackung 6, z.B. wenn diese aus einem für die Insekten 2 in der Verpackung 6 verzehrbaren Material ausgeführt ist, durch die Insekten 2 in der Verpackung 6 verzehrt wird und sich die Verpackung 6 dadurch zumindest teilweise öffnet. Damit müsste die Verpackung 6 nicht einmal vorher geöffnet werden. Somit gelangen die Insekten 2 in das Substrat 4 des Behälters 3. Zudem entfällt das Entfernen der Verpackung aus dem Behälter 3. Die Verpackung 6 kann dabei auch dazu ausgebildet sein, um von den Insekten 2 im Behälter 3 verzehrt zu werden.

In Fig. 4 ist eine bevorzugte Anordnung 10 der erfindungsgemäßen Dosiervorrichtung 1 dargestellt, wobei die Dosiervorrichtung 1 im Bereich eines Fördersystem 9 angeordnet ist und der zumindest eine Behälter 3 auf dem Fördersystem 9 angeordnet ist und mit dem Fördersystem 9 bewegt wird. Beispielsweise kann wie in Fig. 4 dargestellt ein Förderband vorgesehen sein, auf welchem eine Mehrzahl an mit Substrat 4 gefüllten Behältern 3 angeordnet ist. Dabei ist das Förderband dazu ausgebildet, die Behälter 3 in eine Förderrichtung (mit einem Pfeil angedeutet) zu bewegen. Die Robotereinheit 7 füllt der Reihe nach (in Förderrichtung) die Behälter 3 mit Insekten 2 aus den Verpackungen 6. Wie in Fig. 4 beispielhaft dargestellt ist der in Förderrichtung vorderste Behälter 3 bereits mit Insekten 2 gefüllt und kann weiters in der Insektenzucht (z.B. zur Einlagerung des Behälters 3) verwendet werden. Die gefüllten Behälter 3 werden vom Fördersystem 9 weiterbewegt und gelangen dadurch in einen anderen Bereich einer Insektenzuchtanlage, beispielsweise in eine Lagerhalle, wobei die gefüllten Behälter 3 in einem Regallager übereinandergestapelt und für einen vorgegebene Zeitraum gelagert werden, wobei die Insekten 2 in den Behältern 3 weiterwachsen.

## Patentansprüche

1. Verfahren zum Einfüllen von Insekten (2) in zumindest einen Behälter (3) mit einer Dosiervorrichtung (1), wobei die Insekten (2) in einem gleichen bestimmten Wachstumsstadium, insbesondere als Larven, oder in unterschiedlichen bestimmten Wachstumsstadien sind, wobei der zumindest eine Behälter (3) mit einem Substrat (4) gefüllt wird, welches vorgegebene Eigenschaften aufweist, um ein Wachstum der Insekten (2) in dem zumindest einen Behälter (3) zu fördern, wobei die Insekten (2) in zumindest einer Lagereinheit (5) der Dosiervorrichtung (1) gelagert werden, **dadurch gekennzeichnet, dass** die Insekten (2) in einer vorgegebenen Menge in einer Verpackung (6) verpackt sind und die Verpackung (6) mit den Insekten (2) in der vorgegebenen Menge in der zumindest einen Lagereinheit (5) gelagert wird, wobei die vorgegebene Menge der Insekten (2) in einem bekannten Verhältnis auf die vorgegebenen Eigenschaften des Substrats (4) im zumindest einen Behälter (3) abgestimmt ist, **und dass** die Verpackung (6) mit den Insekten (2) in der vorgegebenen Menge von einer Robotereinheit (7) der Dosiervorrichtung (1) aus der zumindest einen Lagereinheit (5) entnommen wird und die Robotereinheit (7) die Verpackung (6) mit den Insekten (2) in der vorgegebenen Menge zum zumindest einen Behälter (3) bewegt und die Robotereinheit (7) die Insekten (2) in der vorgegebenen Menge aus der Verpackung (6) in das Substrat (4) des zumindest einen Behälters (3) direkt füllt, indem die Robotereinheit (7) die Verpackung (6) öffnet und die Insekten (2) in der vorgegebenen Menge in den zumindest einen Behälter (3) füllt, oder indirekt füllt, indem die Robotereinheit (7) die Verpackung (6) mit den Insekten (2) in der vorgegebenen Menge in den zumindest einen Behälter (3) legt.

2. Verfahren nach Anspruch 1, **dadurch gekennzeichnet, dass** bevor oder gleichzeitig oder nachdem die Insekten (2) in der vorgegebenen Menge in den zumindest einen Behälter (3) gefüllt werden, der zumindest eine Behälter (3) mit dem Substrat (4) befüllt wird.

3. Verfahren nach Anspruch 1 oder 2, **dadurch gekennzeichnet, dass** die Robotereinheit (7) das Substrat (4) in den zumindest einen Behälter (3) füllt.

4. Verfahren nach einem der Ansprüche 1 bis 3, **dadurch gekennzeichnet, dass** die Verpackung (6) zusätzlich zu der vorgegebenen Menge an Insekten (2) mit einem Substrat (8) gefüllt ist, wobei das Substrat (8) vorgegebene Eigenschaften aufweist.

5. Verfahren nach Anspruch 4, **dadurch gekennzeichnet, dass** die vorgegebene Menge an Insekten (2) und das Substrat (8) in der Verpackung (6) gleichmäßig vermengt sind.

## Claims

1. A method for filling insects (2) into at least one container (3) with a dosing device (1), wherein the insects (2) are in a same specific growth stage, in particular as larvae, or in different specific growth stages, wherein the at least one container (3) is filled with a substrate (4), which has predetermined properties to promote growth of the insects (2) in the at least one container (3), wherein the insects (2) are stored in at least one storage unit (5) of the dosing device (1), **characterized in that** the insects (2) are packed in a predetermined quantity in a package (6) and the package (6) with the insects (2) in the predetermined quantity is stored in the at least one storage unit (5), wherein the predetermined quantity of insects (2) is matched in a known ratio to the predetermined properties of the substrate (4) in the at least one container (3), **and in that** the package (6) with the insects (2) in the predetermined quantity is removed by a robot unit (7) of the dosing device (1) from the at least one storage unit (5) and the robot unit (7) moves the package (6) with the insects (2) in the predetermined quantity to the at least one container (3) and the robot unit (7) directly fills the insects (2) in the predetermined quantity from the package (6) into the substrate (4) of the at least one container (3), by the robot unit (7) opening the package (6) and filling the insects (2) in the predetermined quantity into the at least one container (3), or indirectly, by the robot unit (7) laying the package (6) with the insects (2) in the predetermined quantity into the at least one container (3).

2. The method according to claim 1, **characterized in that** before or simultaneously or after the insects (2) in the predetermined quantity are filled into the at least one container (3), the at least one container (3) is filled with the substrate (4).

3. The method according to any of claims 1 or 2, **characterized in that** the robot unit (7) fills the substrate (4) into the at least one container (3).

4. The method device (1) according to any of claims 1 to 3, **characterized in that** the package (6) is filled with a substrate (8) in addition to the predetermined quantity of insects (2), wherein the substrate (8) has predetermined properties.

5. The method device (1) according to claim 4, **characterized in that** the predetermined quantity of insects (2) and the substrate (8) are evenly mixed in the package (6).

## Revendications

1. Procédé pour l'introduction d'insectes (2) dans au moins un récipient (3) et comportant un dispositif de dosage (1), dans lequel les insectes (2) sont à un même stade de croissance déterminé, en particulier au stade larvaire, ou à différents stades de croissance déterminés, dans lequel l'au moins un récipient (3) est rempli d'un substrat (4) présentant des propriétés prédéfinies afin de favoriser la croissance des insectes (2) dans l'au moins un récipient (3), dans lequel les insectes (2) sont stockés dans au moins une unité de stockage (5) du dispositif de dosage (1), **caractérisé en ce que** les insectes (2) sont conditionnés en une quantité prédéfinie dans un emballage (6) et l'emballage (6) comportant les insectes (2) en quantité prédéfinie est stocké dans l'au moins une unité de stockage (5), dans lequel la quantité prédéfinie d'insectes (2) concorde, selon un rapport connu, avec les propriétés prédéfinies du substrat (4) dans l'au moins un récipient (3), **et en ce que** l'emballage (6) comportant les insectes (2) en quantité prédéfinie est prélevé de l'au moins une unité de stockage (5) par une unité robotique (7) du dispositif de dosage (1), et l'unité robotique (7) déplace l'emballage (6) comportant les insectes (2) en quantité prédéfinie vers l'au moins un récipient (3) et l'unité robotique (7) remplit d'insectes (2) en quantité prédéfinie le substrat (4) de l'au moins un récipient (3) à partir de l'emballage (6), dans lequel l'unité robotique (7) ouvre l'emballage (6) et remplit, ou remplit indirectement, d'insectes (2) en quantité prédéfinie l'au moins un récipient (3), dans lequel l'unité robotique (7) dépose l'emballage (6) comportant les insectes (2) en quantité prédéfinie dans l'au moins un récipient (3).

2. Procédé selon la revendication 1, **caractérisé en ce qu'**avant, pendant ou après le remplissage de l'au moins un récipient (3) d'insectes (2) en quantité prédéfinie, l'au moins un récipient (3) est rempli de substrat (4).

3. Procédé selon la revendication 1 ou 2, **caractérisé en ce que** l'unité robotique (7) remplit l'au moins un récipient (3) de substrat (4).

4. Procédé selon l'une des revendications 1 à 3, **caractérisé en ce que** l'emballage (6) est rempli, en outre de la quantité prédéfinie d'insectes (2), d'un substrat (8), dans lequel le substrat (8) présente des propriétés prédéfinies.

5. Procédé selon la revendication 4, **caractérisé en ce que** la quantité prédéfinie d'insectes (2) et le substrat (8) sont mélangés de manière régulière dans l'emballage (6).
